# EUROPEAN PATENT APPLICATION

(11) **EP 3 791 819 A1**
(43) Date of publication of application: **17.03.2021**
(21) Application number: 20195726.3
(22) Date of filing: 11.09.2020
(51) Int. Cl.: A61B 18/14, A61B 34/00

(54) **GRAPHICAL USER INTERFACE FOR AN MULTI-ELECTRODE ABLATION SYSTEM**

(30) Priority: 12.09.2019 US 201962899605 P; 08.09.2020 US 202017014716
(71) Applicant: Biosense Webster (Israel) Ltd, Yokneam, 2066717 (IL)
(72) Inventor: ROSENBERG, Avigdor, 2066717 Yokneam (IL); ADAWI, Eid, 2066717 Yokneam (IL)
(74) Representative: Small, Gary James

(57) **Abstract**

An ablation catheter with an expandable balloon or a basket, a plurality of electrodes disposed on the surface, and a graphical user interface (GUI) of a computer system is disclosed. The GUI includes a plurality of electrode representations, a first sector color coded plot of at least one of the plurality of electrodes, a second sector color coded plot of at least one of the plurality of electrodes, a third sector color coded plot of at least one of the plurality of electrodes, and a processor configured to receive data on the inputs based on at least one of the plurality of electrodes, change an appearance of at least one of the first, second sector, and the third sector based on the received data, and update the color coded plot of the first input.

## Description

### CROSS REFERENCE TO RELATED APPLICATIONS

This application claims the benefit of U.S Provisional Application No. 62/899,605, filed September 12, 2019, which is incorporated by reference as if fully set forth.

This application also relates to U.S. Provisional Patent Application No. 62/869,516 filed July 1, 2019 (Ref. No. BIO6141USPSP1) and to U.S. Design Application 29/693,273, filed May 31, 2019 (Ref. No. BIO6141USDP1), which are all incorporated by reference as if fully set forth herein.

### FIELD OF INVENTION

The subject matter disclosed herein relates to ablation systems, including systems that include a multi-electrode catheter capable of ablating cardiac tissue and a graphical user interface system to assist in performing the ablation including circular multi-electrode catheters, such as balloon or basket catheters.

### BACKGROUND

Ablation of cardiac tissue has been used to treat cardiac arrhythmias. Ablative energies are typically provided to cardiac tissue by a tip portion which can deliver ablative energy to the tissue to be ablated. Some of these catheters administer ablative energy from various electrodes including three-dimensional structures, e.g. balloon or basket-shaped multi-electrode ablation catheters. Ablative procedures incorporating such catheters may be visualized using imaging modalities, e.g., fluoroscopy, or 3D navigation and mapping systems.

### SUMMARY

An ablation system comprises a catheter including an expandable balloon disposed thereon, the expandable balloon including a surface and a plurality of electrodes disposed on the surface and a graphical user interface (GUI). The GUI comprises a main sector including an image of the expandable balloon comprising a plurality of electrode representations including a first-electrode representation, a first sector including a color coded plot of a first input for at least one of the plurality of electrodes, a second sector including a color coded plot of a second input for at least one of the plurality of electrodes, a third sector including a color coded plot of a third input for at least one of the plurality of electrodes, and a processor configured to receive data on at least one of the first input, the second input, and the third input based on a status of at least one of the plurality of electrodes, change an appearance of at least one of the first sector, the second sector, and the third sector based on the received data corresponding to the first input, the second input, and the third input, update the color coded plot of the first input for at least one of the plurality of electrodes, the second input for at least one of the plurality of electrodes, and the third input for at least one of the plurality of electrodes.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1 is a schematic illustration of an invasive medical procedure;
Figure 2 is a top view of a catheter with a balloon in an expanded state, in use with a lasso catheter;
Figure 3 is a perspective view of a balloon of Figure 2, along with the lasso catheter;
Figure 4 is a side view of a distal end of the catheter of Figure 2 deployed in the region of a pulmonary vein and its ostium;
Figure 5 is a representation of a graphical user interface (GUI) display;
Figure 6A illustrates an exemplary two-dimensional display with an electrode representation before ablation;
Figure 6B illustrates another exemplary two-dimensional display with an electrode representation;
Figure 6C illustrates an exemplary bar graph for the GUI display of Figure 5;
Figure 6D illustrates an on-screen selection of preferences for the balloon alignment indicators, used before ablation, described herein.
Figure 7A illustrates an exemplary two-dimensional display with an electrode representation during ablation;
Figure 7B illustrates another exemplary two-dimensional display with an electrode representation during ablation;
Figure 8A is an exemplary two-dimensional display with an electrode representation post ablation;
Figure 8B illustrates a bar graph associated with the two-dimensional display of Figure 8A;
Figure 8C illustrates another exemplary two-dimensional display with an electrode representation post ablation;
Figure 8D illustrates a bar graph associated with the two-dimensional display of Figure 8C;
Figure 8E illustrates on on-screen selection of preferences for the balloon alignment, used before ablation, and session review indicators, used during ablation and/or post-ablation, described herein;
Figure 9 represents an illustration of a radar-plot of baseline impedance that may be provided in a sector of GUI display;
Figure 10 represents an illustration of a radar-plot of baseline impedance and real-time impedance during and/or at end of ablation, that may be provided in a sector of GUI display;
Figure 11 represents an illustration of a circle bar plot of baseline impedance that may be provided in a sector in GUI display;
Figure 12 represents an illustration of circle bar plot of baseline impedance and real-time impedance during and/or at end of ablation, that may be provided in second sector for display in GUI display;
Figure 13 represents an illustration of a circle bar plot of baseline temperature that may be provided in second sector for display in GUI display;
Figure 14 represents an illustration of circle bar plot of baseline temperature and real-time temperature during and/or at end of ablation, that may be provided in second sector for display in GUI display; and
Figure 15 illustrates a method 1500 of using a graphical user interface (GUI) of a computer system.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values such as ±10% of the recited value, as an example. In addition, as used herein, the terms "patient," "host," "user," and "subject" refer to any human or animal subject and are not intended to limit the systems or methods to human use, although use of the subject invention in a human patient represents a preferred embodiment.

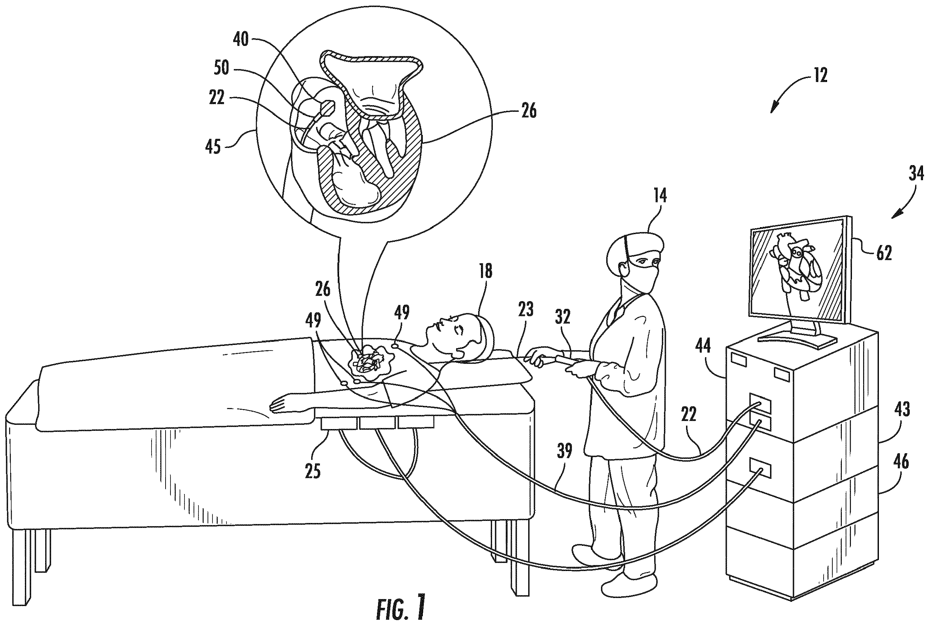

Figure 1 is a schematic, pictorial illustration of an electro-anatomical mapping system 12, in accordance with an embodiment of the present invention. A physician 14 navigates a catheter 40 (for example, made by Biosense-Webster), seen in detail in inset 45, to a target location in a heart 26 of a patient 18, by manipulating shaft 22, using a manipulator 32 near the proximal end of the catheter, and/or deflection from a sheath 23. In the embodiment seen in inset 45, physician 14 uses catheter 40 to perform electro-anatomical mapping of a cardiac chamber. Mapping system 12 may further utilize external electrodes 49.

Catheter 40 is inserted in a folded configuration, through sheath 23, and only after catheter 40 is extracted from sheath 23 does catheter 40 regain its intended functional shape.

Catheter 40 incorporates a magnetic sensor 50, seen in inset 45, at the distal edge of shaft 22 (i.e., at the proximal edge of balloon/basket catheter 40).

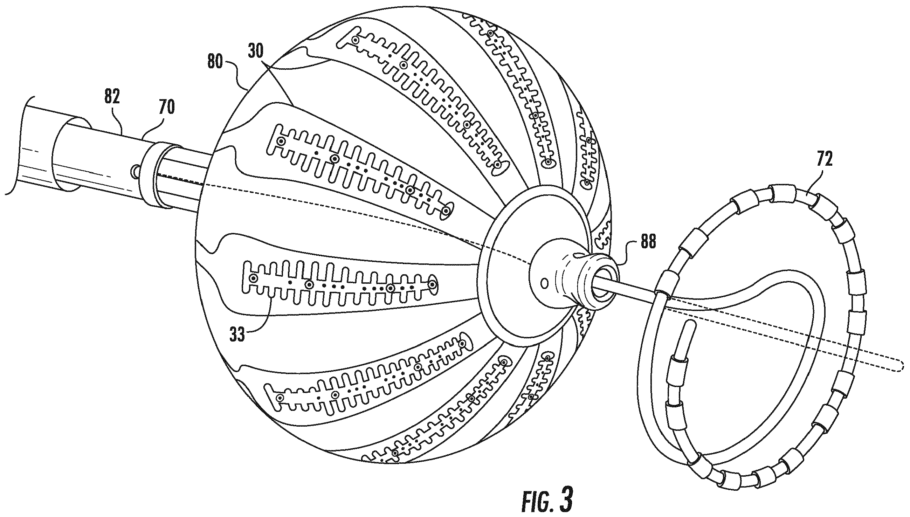

Catheter 40 further comprises multiple expandable spines 30 (shown in Figure 3), which may be mechanically flexible, to each of which are coupled one or more devices 33 (shown in Figure 3). Devices 33 may be of numerous types, such as sensing-electrodes, ablation-electrodes, ultrasound transducers, contact force sensors, irrigation ports, temperature sensors, and others. Magnetic sensors 50 and devices 33 are connected by wires running through shaft 22 to various driver circuitries in a console 34.

In some embodiments, system 12 comprises a magnetic-sensing subsystem to estimate an ellipticity of catheter 40 inside a cardiac chamber of heart 26 by estimating the elongation of catheter 40. Patient 18 is placed in a magnetic field generated by a pad containing magnetic field generator coils 25, which are driven by unit 43. The magnetic fields generated by coils 25 generate signals in sensors 50, which are indicative of position and/or direction. The generated signals are transmitted to console 34 and become corresponding electrical inputs to a processor 46. The processor uses the signals to calculate the elongation of catheter 40, and to estimate ellipticity from the calculated elongation.

The method of position and/or direction sensing using external magnetic fields and magnetic sensors, such as 50, is implemented in various medical applications, for example, in the CARTO® system, produced by Biosense-Webster, and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference.

Processor 46, typically a general-purpose computer, is further connected via suitable front end and interface circuits 44, to receive signals from surface-electrodes 33. Processor 46 is connected to surface-electrodes 33 by wires running through a cable 39 to the chest of patient 18.

In an embodiment, processor 46 additionally receives various spatial and electrophysiological signals via an electrical interface 44, and uses the information contained in these signals to construct an electro-anatomical map of the cavity. During and/or following the procedure, processor 46 may display electro-anatomical map on a display 62.

Processor 46 is typically programmed in software to carry out the functions described herein. In particular, processor 46 runs a dedicated algorithm that enables processor 46 to perform the disclosed steps, as described below.

The example illustration shown in Fig. 1 is chosen purely for the sake of conceptual clarity. Fig. 1 shows only elements related to the disclosed techniques for the sake of simplicity and clarity. System 12 typically comprises additional modules and elements that are not directly related to the disclosed techniques, and thus are intentionally omitted from Fig. 1 and from the corresponding description. The elements of system 12 and the methods described herein may be further applied, for example, to control an ablation of tissue of heart 26.

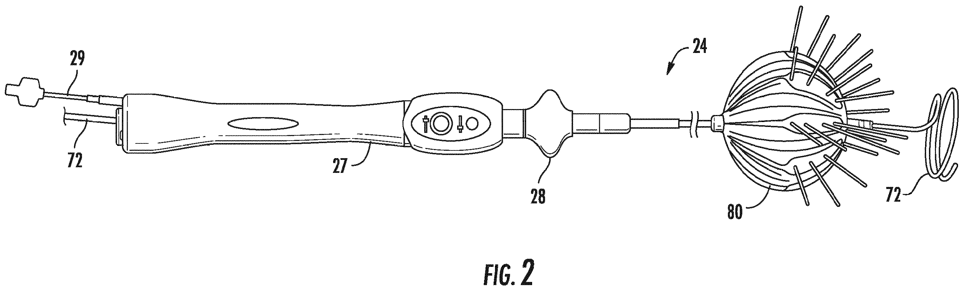

Figure 2 is a schematic perspective view of the diagnostic/therapeutic catheter 24 in in which balloon 80 is in an expanded configuration. Catheter 24 may include a handle 27, a plunger 28, and irrigation tubing 29. An irrigation fluid (e.g., saline) may be introduced or pumped through tubing 29 and ultimately into balloon 80 by a pump connected to a source of the irrigation fluid.

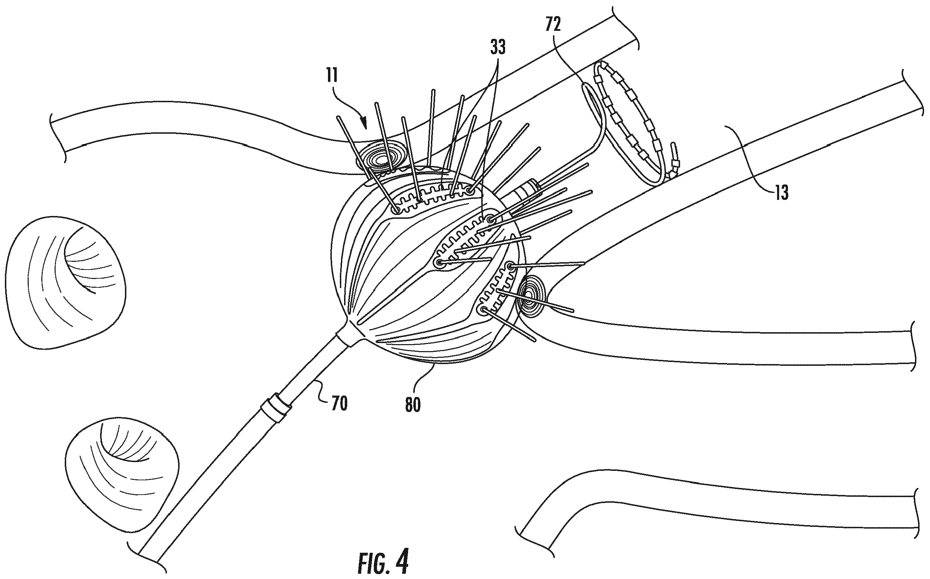

With further reference to Figures 3 and 4, where the therapeutic catheter 24 is used to ablate an ostium 11 of a lumen, such as a pulmonary vein 13, the therapeutic catheter 24 may be supported by a tubular shaft 70 having a proximal shaft portion 82 and a distal shaft end 88. The shaft 70 includes a hollow central tube (not shown), which permits another catheter to pass therethrough and past the distal shaft end 88, such as a lasso catheter 72. The lasso catheter 72 may be inserted into the pulmonary vein to position the therapeutic catheter 24 correctly with respect to the ostium prior to ablation of the ostium. Any catheter used in conjunction with the therapeutic catheter may have features and functions, including, for example, pressure sensing, ablation, diagnostic, e.g., navigation and pacing.

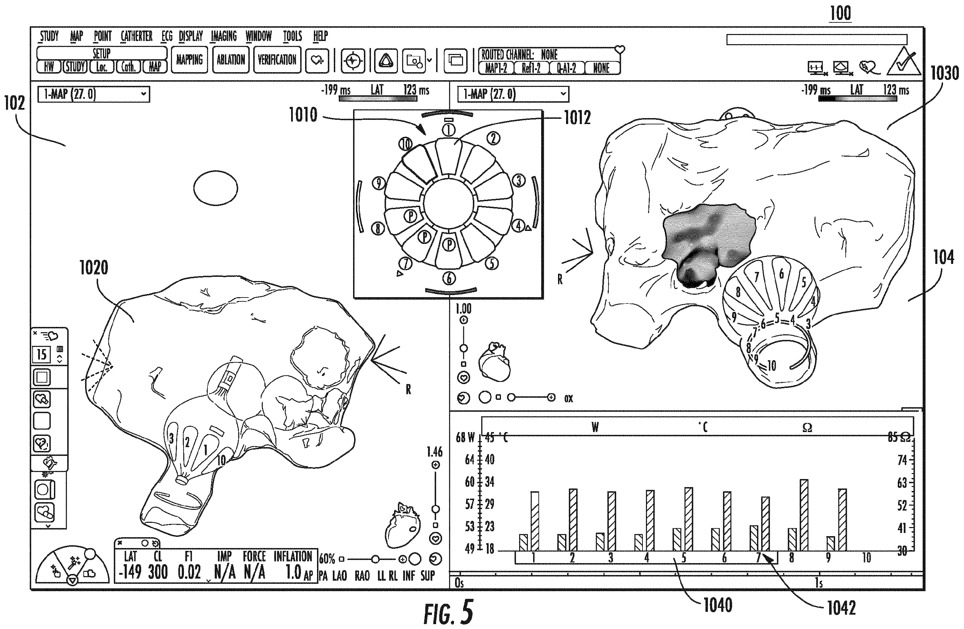

Screen 62 or an additional iteration thereof, may be used to display one or more displays of a graphical user interface (GUI), which may assist medical professional 14 to receive information therefrom to conduct an ablation procedure using therapeutic catheter 24. Representations of exemplary displays and sections of the GUI are reflected in Figures 5 - 23. Medical professional 14 may provide certain inputs to processor 46 via the various GUI displays. The various GUI displays may also be used to guide medical professional 14 through the procedure by providing, e.g., display of relevant pre-ablation, ablation and post-ablation data, recommendations, notifications, and alerts. Accordingly, each display may correspond to a distinct step or segment of the ablation procedure. As such, the GUI displays may be configured to permit medical professional 14 to make inputs to processor 46 relevant to the step or segment of the ablation procedure, in order to control his preferences of the data displayed on the screen. Furthermore, based on determinations made by processor 46, processor 46 may modify the displays according to the relevant step of the procedure, e.g. pre-ablation, during ablation, post-ablation.

A first exemplary representation of a GUI display 100 is reflected in Figure 5. Display 100 may include a first sector 102 and second sector 104. First sector 102 and second sector 104 may be used to show different information corresponding to a procedure. GUI 100 includes a two-dimensional display with electrodes representation 1010 included thereon. The electrodes representation 1010 may provide an indication of all electrodes and context-based data according to the current activity.

In the context before ablation, the physician 14 aligns the balloon with the antrum of the vein to get a good contact with the tissue. Physician 14 verifies the electrodes selected for ablation and the electrodes selected as posterior, at the generator (at which less ablation energy will be applied by the generator). Representation 1010, before ablation, may illustrate the different types of data relevant before ablation, such as which electrodes are in contact with the tissue using balloon alignment indicators, the electrodes selected for ablation at the generator, the electrodes selected as posterior at the generator, and the electrodes with bad readings, e.g., a disconnected electrode.

In the context that occurs during ablation, the physician 14 monitors the impedance drops and temperature rise of the ablating electrodes, as well as the state of the electrodes, i.e., the electrodes that are ablating and the electrodes that are not. Representation 1010, during ablation, may illustrate the different types of data relevant during ablation, such as the ablating electrodes and the electrodes not ablating, provide a dynamic display value of impedance drop and/or temperature rise, the electrodes selected as posterior at the generator, and the electrodes with bad readings, e.g., a disconnected electrode.

Representation 1010 may be used for display of other types of information, such as display of time from start of ablation, which may be shown in the center of the representation 1010, for example.

In the context that occurs after ablation, the physician 14 reviews the values of impedance drop and temperature rise to decide where repeated ablation might be needed. Impedance drop and temperature rise values are considered to be good surrogates for quality of ablation. Representation 1010, after ablation, may be used to review the parameters of impedance drop or temperature rise of ablation sessions illustrating a display value of impedance drop or temperature rise, the electrodes selected as posterior at the generator, and the electrodes turned off and did not ablate.

Electrode representation 1010 may provide feedback to professional 14 by providing an outline 1012 of each electrode. Outline 1012 may provide a color indication, such as by turning white or having a white contour, to indicate that the configuration of the electrodes provides a probability that good tissue contact exists and the likelihood of a successful ablation is configured. When all of the electrodes in the electrode representation 1010 are depicted with outline 1012 indicating that good tissue contact exists for all electrodes, the balloon positioning may be indicated as good. The status of the electrodes may also be included within the electrode representation 1010 including active/non-active, posterior and touch/no-touch. The status indicator enables the professional 14 to monitor selection made on the RF generator by the generator operator.

GUI 100 further includes a dual-depiction 1020, 1030 of the image of the balloon within the pulmonary vein in first sector 102 and second sector 104, respectively, as set forth above with respect to at least Figure 4. Each of the electrodes in the dual-depiction 1020, 1030 may be identified. Further, a camera may be provided within the balloon in order to provide images from "inside" the ablation. Images from the camera may be provided within a sector of GUI 100.

An additional portion of GUI 100 includes a bar graph 1040. Bar graph 1040 may include an indication 1042 of the balloon positioning for each electrode. In this case, the indication 1042 is included below the bar graph 1040 where the electrode numbers are highlighted in white to indicate good tissue contact. Bar graph 1040 may simultaneously depict temperature and impedance. This indication conveys to the professional 14 that if there is a white highlight for indication 1042, the criteria for successful ablation is met and if there is a black highlight for indication 1042, the criteria for successful ablation is not met. Once all electrodes are highlighted for indication 1042 to indicate successful ablation criteria are met, the balloon positioning is good.

Bar graph 1040 may show the initial impedance values for each ablating electrode and the current impedance, as well as the initial temperature and the current temperature. Colors may be used to delineate the various types of information.

All electrodes 33 that are active, e.g., electrodes corresponding to electrode icons may optionally also have their color changed, e.g., to a gold color, and those deactivated identified as well. Display 100 may also provide indications of physical attributes, e.g., current, power, temperature and impedance, for each active electrode. Status bars may be provided indicating the instantaneous impedance at each electrode.

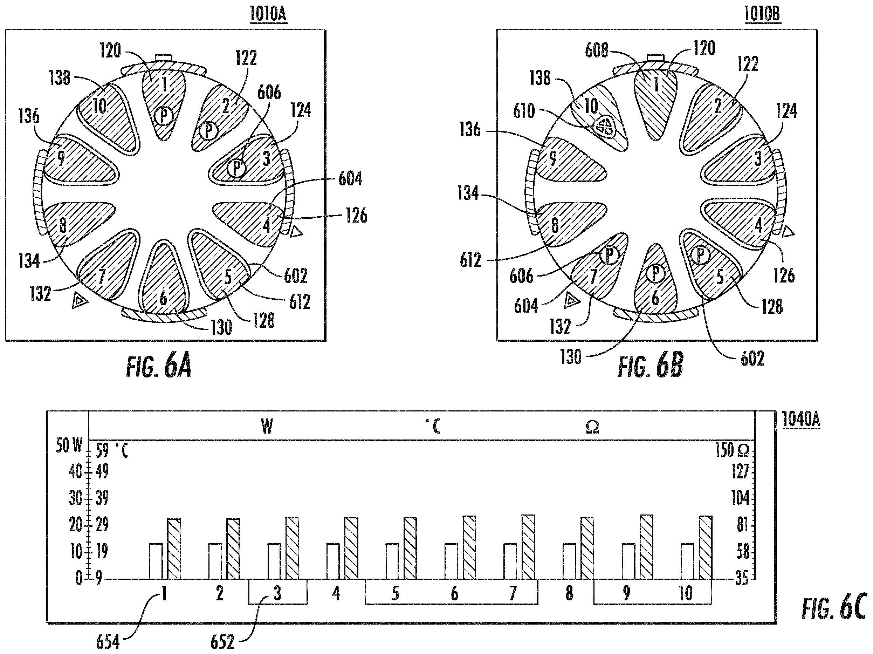

An exemplary two-dimensional display with an electrode representation before ablation 1010A included thereon is illustrated in Figure 6A. Illustration 1010A includes a depiction of each of the electrodes 1-10, with a first electrode depiction 120, a second electrode depiction 122, a third electrode depiction 124, a fourth electrode depiction 126, a fifth electrode depiction 128, a sixth electrode depiction 130, a seventh electrode depiction 132, an eighth electrode depiction 134, a ninth electrode depiction 136 and a tenth electrode depiction 138. Overlaid on the electrode depiction is an initial - before ablation - information. As would be understood from the description below, all electrodes need not contain the numeral indication as only those within range, or those outside of the range may include indications.

Representation 1010A includes an indication of the electrodes which are in touch with the tissue indicated with a white contour 602, by example. In contrast, electrode 4 is not indicated with a white contour, instead a black contour 604, or lack of contour is used. Specifically, in the example electrodes 3,5,6,7,9, 10 are represented using the white contour 602 to indicate touch with the tissue, while electrodes 1, 2,4, 8 are represented using the black contour 604. All of the electrodes 1-10 are selected for ablation and are indicated as being selected in a yellow color 612, by example. Electrodes 1,2,3 are selected as posterior and indicated by being marked with a "P" marking 606, by example, although any other distinctive marking may be used.

Another exemplary two-dimensional display with an electrode representation 1010B included thereon is illustrated in Figure 6B. Illustration 1010B includes a depiction of each of the electrodes 1-10, with a first electrode depiction 120, a second electrode depiction 122, a third electrode depiction 124, a fourth electrode depiction 126, a fifth electrode depiction 128, a sixth electrode depiction 130, a seventh electrode depiction 132, an eighth electrode depiction 134, a ninth electrode depiction 136 and a tenth electrode depiction 138. Overlaid on the electrode depiction is an initial - before ablation - information. As would be understood from the description below, all electrodes need not contain the numeral indication as only those within range, or those outside of the range may include indications.

Representation 1010B includes an indication that electrode 1 unselected and indicated as such by being marked gray 608, by example. Electrode 10 is marked with an "X" 610, by example, to indicate that it has abnormal readings, i.e., likely disconnected. As described in Figure 6B, electrodes 5, 6, 7 are selected as posterior and indicated by being marked with a "P" marking 606, by example. Electrodes 2, 3, 4, 5 indicated with a white contour 602, by example, to indicate contact with the tissue. Electrodes 6, 7, 8, 9 are indicated with a black contour 604 to indicate no tissue contact. Electrodes 2, 3, 4, 5, 6, 7, 8, 9 are represented as being selected in a yellow color 612, by example

Figure 6C illustrates an exemplary bar graph 1040A. Graph 1040A may be used as an additional form of indication for the balloon alignment indicators, for example by white background 652 / black background 654 under the electrode numbers to indicate tissue contact/no tissue contact.

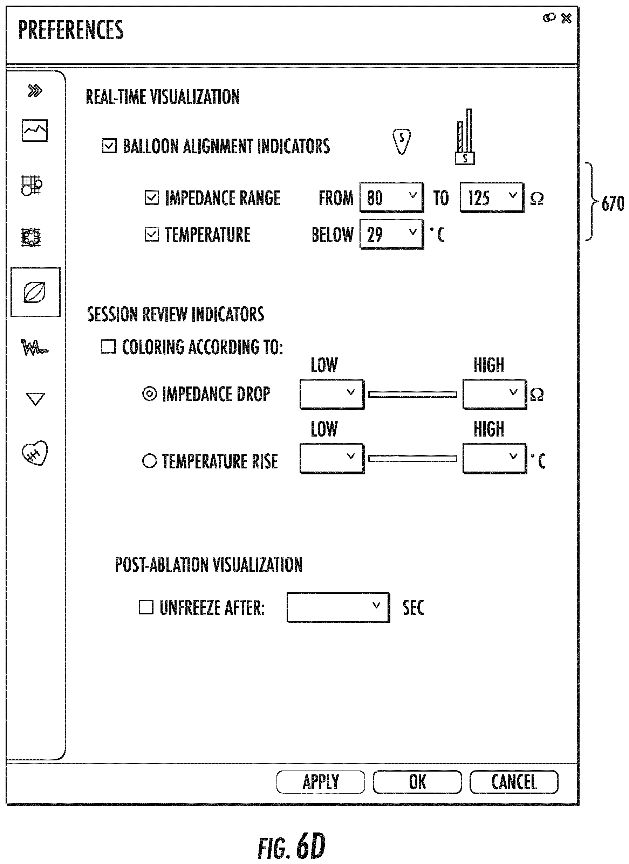

Figure 6D illustrates on on-screen selection of preferences for the balloon alignment indicators described herein. For example, a preference 670 may be used to select if the indicators are based on impedance and/or temperature measurements by the balloon electrodes. If the impedance is within a certain range of values, and/or the temperature is below a certain value, it is a good surrogate for contact of the electrode with the tissue. The color setting may be set to indicate color tones.

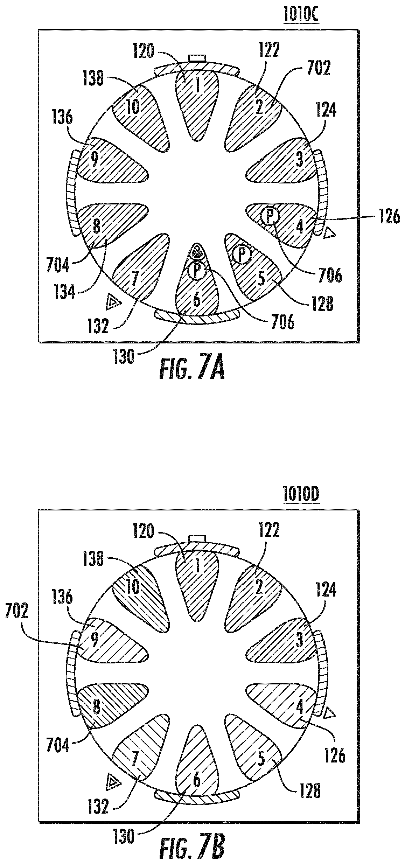

An exemplary two-dimensional display with an electrode representation during ablation 1010C included thereon is illustrated in Figure 7A. Illustration 1010C includes a depiction of each of the electrodes 1-10, with a first electrode depiction 120, a second electrode depiction 122, a third electrode depiction 124, a fourth electrode depiction 126, a fifth electrode depiction 128, a sixth electrode depiction 130, a seventh electrode depiction 132, an eighth electrode depiction 134, a ninth electrode depiction 136 and a tenth electrode depiction 138. Overlaid on the electrode depiction is an on-going - during ablation - information. As would be understood from the description below, all electrodes need not contain the numeral indication as only those within range, or those outside of the range may include indications.

Representation 1010C includes an indication of the electrodes which are marked to indicate ablation 702. Such a marking may include a color such as red, for example. Electrodes 1, 2,3, 4, 5, 9, 10 are indicating ablation in the example. Electrodes marked gray 704 are turned off. In the example, this includes electrodes 7, 8. Electrodes marked with an "X" 706 have readings out of limits. In the example, this includes electrode 6. Electrodes marked with "P" 708 indicate that selection as posterior at the generator. In the example, posterior selection includes electrodes 4, 5, 6.

Another exemplary two-dimensional display with an electrode representation during ablation 1010D included thereon is illustrated in Figure 7B. Illustration 1010D includes a depiction of each of the electrodes 1-10, with a first electrode depiction 120, a second electrode depiction 122, a third electrode depiction 124, a fourth electrode depiction 126, a fifth electrode depiction 128, a sixth electrode depiction 130, a seventh electrode depiction 132, an eighth electrode depiction 134, a ninth electrode depiction 136 and a tenth electrode depiction 138. Overlaid on the electrode depiction is an on-going - during ablation - information. As would be understood from the description below, all electrodes need not contain the numeral indication as only those within range, or those outside of the range may include indications.

Representation 1010D includes dynamic coloring of the electrodes on the 2D view, according to the values of impedance drop. Alternatively, the coloring may be according to values of temperature rise. In the example, the larger the value, the darker the color shade of the electrode. Electrodes having some shade of color are indicated as ablating 702. Electrodes not ablating may be marked grey (same as 704), electrodes out of range may be marked by "X" (same as 706), and by "P" for posterior (same as 708, as described above).

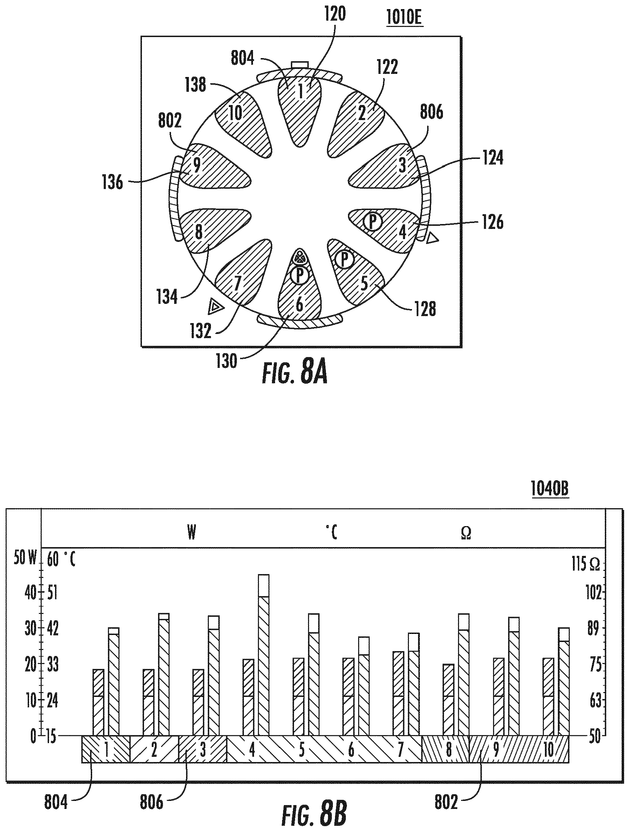

An exemplary two-dimensional display with an electrode representation post ablation 1010E included thereon is illustrated in Figure 8A. Illustration 1010E includes a depiction of each of the electrodes 1-10, with a first electrode depiction 120, a second electrode depiction 122, a third electrode depiction 124, a fourth electrode depiction 126, a fifth electrode depiction 128, a sixth electrode depiction 130, a seventh electrode depiction 132, an eighth electrode depiction 134, a ninth electrode depiction 136 and a tenth electrode depiction 138. Overlaid on the electrode depiction is a post-ablation (i.e. ablation session review) information. As would be understood from the description below, all electrodes need not contain the numeral indication as only those within range, or those outside of the range may include indications.

Representation 1010E includes indications of the electrodes colored by shades of red, proportional to the values of temperature rise and according to the color-scale upper and lower limits, defined in the preferences. By way of example, electrode 136 may be first shade of red 802, electrode 1 may be a second shade of red 804, electrode 3 may be a third shade of red 806. Alternatively, the color shading may be according to the values of impedance drop, or any other relevant data value. In bar graph 1040B of Figure 8B, the same color coding of temperature rises or impedance drop is illustrated. Again, using the example, electrode 136 may be first shade of red 802, electrode 1 may be a second shade of red 804, electrode 3 may be a third shade of red 806.

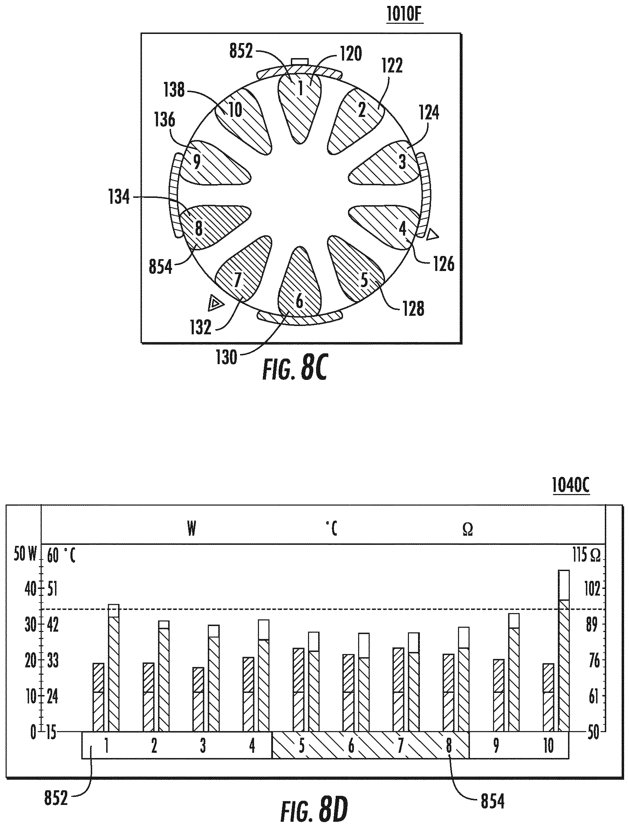

An exemplary two-dimensional display with an electrode representation post ablation 1010F included thereon is illustrated in Figure 8C. Illustration 1010F includes a depiction of each of the electrodes 1-10, with a first electrode depiction 120, a second electrode depiction 122, a third electrode depiction 124, a fourth electrode depiction 126, a fifth electrode depiction 128, a sixth electrode depiction 130, a seventh electrode depiction 132, an eighth electrode depiction 134, a ninth electrode depiction 136 and a tenth electrode depiction 138. Overlaid on the electrode depiction is an on-going, during ablation information, or a post-ablation information. As would be understood from the description below, all electrodes need not contain the numeral indication as only those within range, or those outside of the range may include indications.

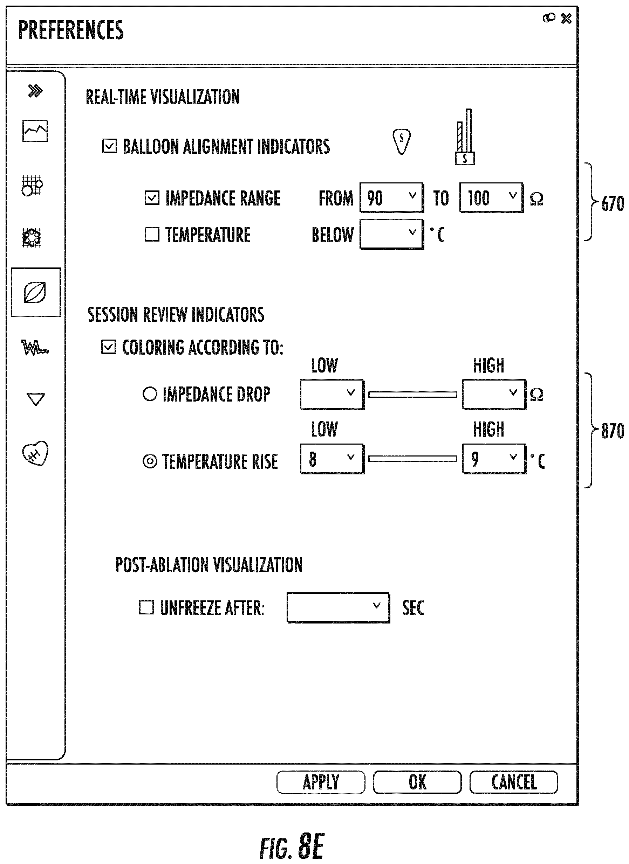

Representation 1010F includes, similar to representation 1010E proportional information, with representation 1010F including indications of the electrodes colored by just two shades (binary coding), according to the thresholds 870 set in the preferences illustrated in Figure 8E. In this example, any electrode which had temperature rise value of 8°C or below is colored pink 852, while any electrode which had temperature rise value of 9°C or above is colored red 854. Pink electrodes 852 includes electrodes 1, 2,3, 4, 9, 10 and red 854 electrodes includes electrodes 5, 6, 7, 8.

In bar graph 1040C of Figure 8D, the same color coding, pink 852 and red 854 is shown. Electrodes which did not ablate will be shown gray. Bar graph 1040 also includes the initial impedance values of the electrodes (in dark green bars) and the final impedance (in bright green bars), as well as the initial temperature (in dark red bars) and the final temperature (in bright red bars).

Figure 8E illustrates on on-screen selection of preferences for the balloon alignment and session review indicators described herein. For example, a preference 870 may be used to select if the indicators during ablation or post-ablation are based on impedance drop or temperature rise values on the balloon electrodes. The color setting 870 may be set to indicate color tones.

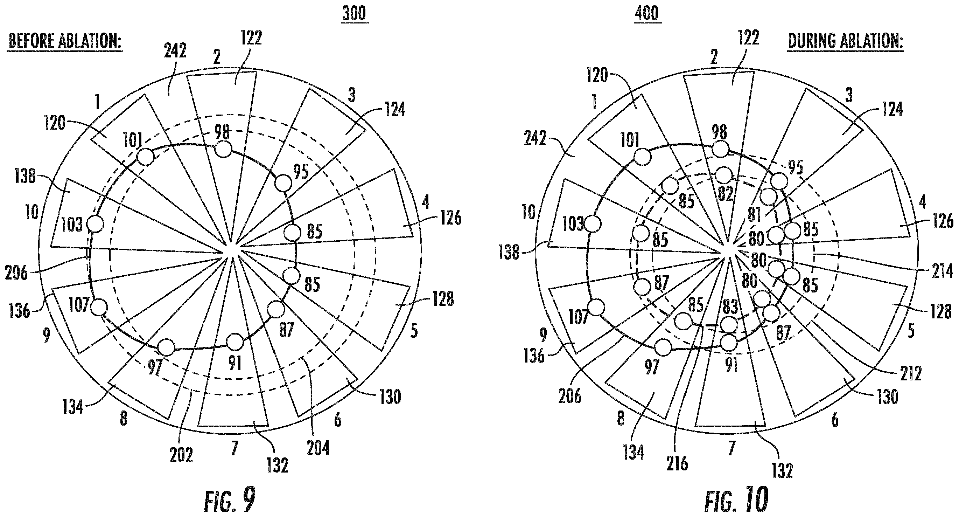

Figure 9 represents an illustration 300 of a radar-plot of baseline impedance that may be provided in a sector of GUI display 100, e.g. instead of 1012, as yet another form of two-dimensional visual presentation of the data values. Further, indications of physical or electrical quantities, e.g., current, temperature or impedance, associated with one or more electrodes 33 may be provided alongside the corresponding electrode icons, in this form of representation, as relevant to the step or segment of the ablation procedure.

Illustration 300 includes a depiction of each of the electrodes 1-10, with a first electrode depiction 120, a second electrode depiction 122, a third electrode depiction 124, a fourth electrode depiction 126, a fifth electrode depiction 128, a sixth electrode depiction 130, a seventh electrode depiction 132, an eighth electrode depiction 134, a ninth electrode depiction 136 and a tenth electrode depiction 138 along with membrane 26 represented by reference numeral 242. Overlaid on the electrode depiction is an initial impedance 206. Initial impedance 206 may include an impedance value for each of the depicted electrodes and a connector connecting the impedance value for the electrode with neighboring depicted electrodes. A numerical indication may be provided to provide an actual number to each depicted point. As would be understood from the description below, all electrodes need not contain the numeral indication as only those within range, or those outside of the range may include indications.

Illustration 300 may include a target range of impedance that may be used before the ablation. Target range of impedance may include a minimum value of target range of impedance 204 and a maximum value of target range impedance 202. As illustrated in illustration 300 both minimum value of target range of impedance 204 and maximum value of target range of impedance 202 are depicted as dotted lines, although any indication may be used. A comparison may be readily performed by professional 14 viewing the display 100 to determine if the initial impedance 206 values for each electrode fall within the target range between the minimum value of target range of impedance 204 and maximum value of target range of impedance 202. When all values of electrodes are within the target range, the professional 14 may conclude that the balloon is well aligned with the target vein. When some part of the balloon is not well aligned and not touching the tissue, the plot would look a-symmetrical/off-center, with the non-touching electrodes, or electrodes too deep in the vein, having values below or above the target range. The initial impedance 206 and the minimum value of target range of impedance 204 and maximum value of target range of impedance 202 may be frozen on the display once the ablation commences.

Figure 10 represents an illustration 400 of a radar-plot of baseline impedance that may be provided in a sector of GUI display 100. Illustration 400 is similar to illustration 300 except for differences described herein. Overlaid on the electrode depiction is the before ablation information of Figure 9. Additionally, overlaid is an impedance 216 measured during the ablation. Impedance 216 may include an impedance value for each of the depicted electrodes and a connector connecting the impedance value for the electrode with neighboring depicted electrodes. A numerical indication may be provided to provide an actual number to each depicted point. As would be understood from the description below, all electrodes need not contain the numeral indication as only those within range, or those outside of the range may include indications.

Illustration 400 may include a target range of impedance that may be desired during the ablation. Target range of impedance may include a minimum value of range of impedance 214 and a maximum value of range impedance 212. As illustrated in illustration 400 both minimum value of range of impedance 214 and maximum value of range of impedance 212 are depicted as dotted lines, although any indication may be used. A comparison may be readily performed by professional 14 viewing the display 100 to determine if the final impedance 216 values, at end of ablation, for each electrode, fall within the target range between the minimum value of range of impedance 214 and maximum value of range of impedance 212. The initial impedance value 206 and the final impedance values 216 may be frozen on the display once the ablation ends. The difference between the initial impedance and the final impedance represents the impedance drop.

Temperature before and during ablation may be similarly depicted based on Figure 9 and Figure 10 as would be understood from the present description.

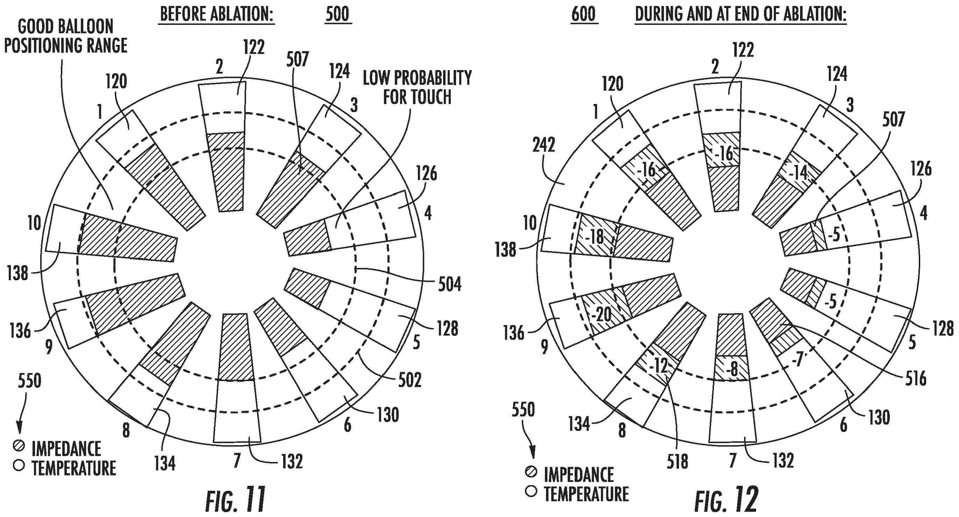

Figure 11 represents an illustration 500 of a circle bar plot of baseline impedance that may be provided in a sector in GUI display 100, e.g. instead of 1012. Illustration 500 is similar to illustration 300 except for differences described herein. Overlaid on the electrode depiction is an initial impedance 507. Initial impedance 507 may include an impedance value for each of the depicted electrodes. A numerical indication (not shown) may be provided to provide an actual number to each depicted point. As would be understood from the description below, all electrodes need not contain the numeral indication as only those within range, or those outside of the range may include indications.

Illustration 500 may include a target range of impedance that may be used before the ablation. Target range of impedance may include a minimum value of target range of impedance 504 and a maximum value of target range impedance 502. As illustrated in illustration 500 both minimum value of target range of impedance 504 and maximum value of target range of impedance 502 are depicted as dotted lines, although any indication may be used. A comparison may be readily performed by professional 14 viewing the display 100 to determine if the initial impedance 507 values for each electrode fall within the target range between the minimum value of target range of impedance 504 and maximum value of target range of impedance 502. The initial impedance 507 and the minimum value of target range of impedance 504 and maximum value of target range of impedance 502 may be frozen on the display once the ablation commences.

Figure 11 includes a toggle 550 to toggle the depiction 500 between impedance display and temperature display. Other relevant data parameters may be presented in a similar manner.

Figure 12 represents an illustration 600 of circle bar plot of baseline impedance that may be provided in second sector 104 for display in GUI display 100. Illustration 600 is similar to illustration 500 except for differences described herein. Overlaid on the electrode depiction is the before ablation information of Figure 11. Additionally, overlaid is impedance 516 measured during the ablation. Impedance 516 may include an impedance value for each of the depicted electrodes. A numerical indication 518 may be provided to provide an actual number to each depicted point. The numerical indication 518 may indicate the change in the impedance 516 from the impedance 507. As would be understood from the description below, all electrodes need not contain the numeral indication as only those within range, or those outside of the range may include indications.

A comparison may be readily performed by professional 14 viewing the display 100 to determine if the initial impedance 507 and impedance 516 are acceptable, as well as the numerical indication 518 between the values. The impedance 516 may be frozen on the display once the ablation ends.

Figure 12 includes a toggle 550 to toggle the depiction 500 between impedance display and temperature display.

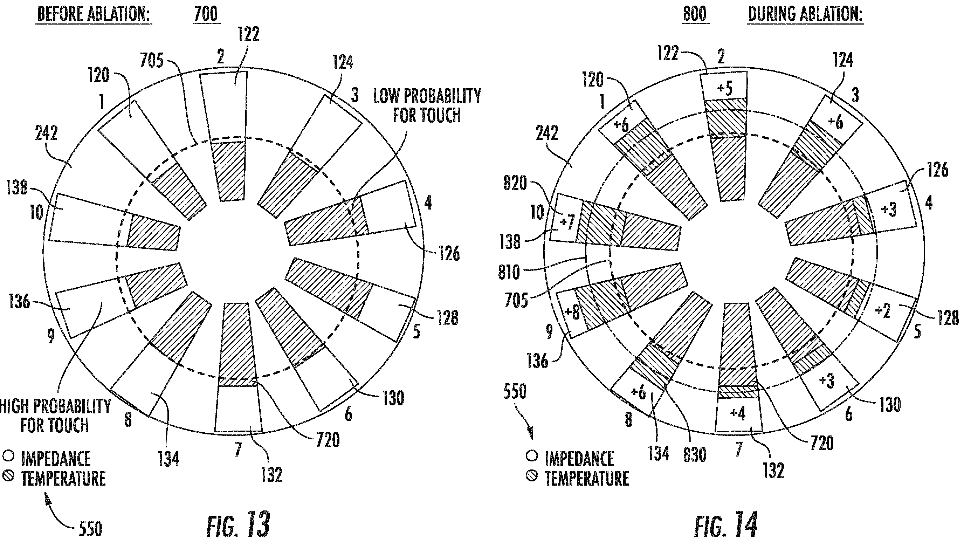

Figure 13 represents an illustration 700 of a circle bar plot of baseline temperature that may be provided in a sector of GUI display 100. Illustration 700 is similar to illustration 300 except for differences described herein. Overlaid on the electrode depiction is an initial temperature 720. Initial temperature 720 may include a temperature value for each of the depicted electrodes. A numerical indication (not shown) may be provided to provide an actual number to each depicted point. As would be understood from the description below, all electrodes need not contain the numeral indication as only those within range, or those outside of the range may include indications.

Illustration 700 may include a maximum target temperature 705 that may be acceptable before the ablation. A comparison may be readily performed by professional 14 viewing the display 100 to determine if the initial temperature 720 values for each electrode fall below the maximum target temperature 705. The initial temperature 720 and maximum target temperature 705 may be frozen on the display once the ablation commences.

Figure 13 includes a toggle 550 to toggle the depiction 700 between impedance display and temperature display. As shown toggle 550 has activated the display between depiction 700 from depiction 500.

Figure 14 represents an illustration 800 of circle bar plot of baseline impedance that may be provided in a sector of GUI display 100. Illustration 800 is similar to illustration 600 except for differences described herein. Overlaid on the electrode depiction is the before ablation information of Figure 13. Additionally, overlaid is a temperature 830 measured during the ablation. Temperature 830 may include a temperature value for each of the depicted electrodes. A numerical indication 820 may be provided to provide an actual number to each depicted point. The numerical indication 820 may indicate the change in the temperature 830 from the temperature 720. A minimum target temperature rise 810 may be indicated to depict for professional 14 if the electrode met the minimum temperature rise. As would be understood from the description below, all electrodes need not contain the numeral indication as only those within range, or those outside of the range may include indications.

A comparison may be readily performed by professional 14 viewing the display 100 to determine if the initial temperature 720 and temperature 830 are acceptable, as well as the numerical indication 820 between the values. The temperature 830 may be frozen on the display once the ablation ends.

Figure 14 includes a toggle 550 to toggle the depiction 800 between impedance display and temperature display. As shown toggle 550 has activated the display between depiction 800 from depiction 600.

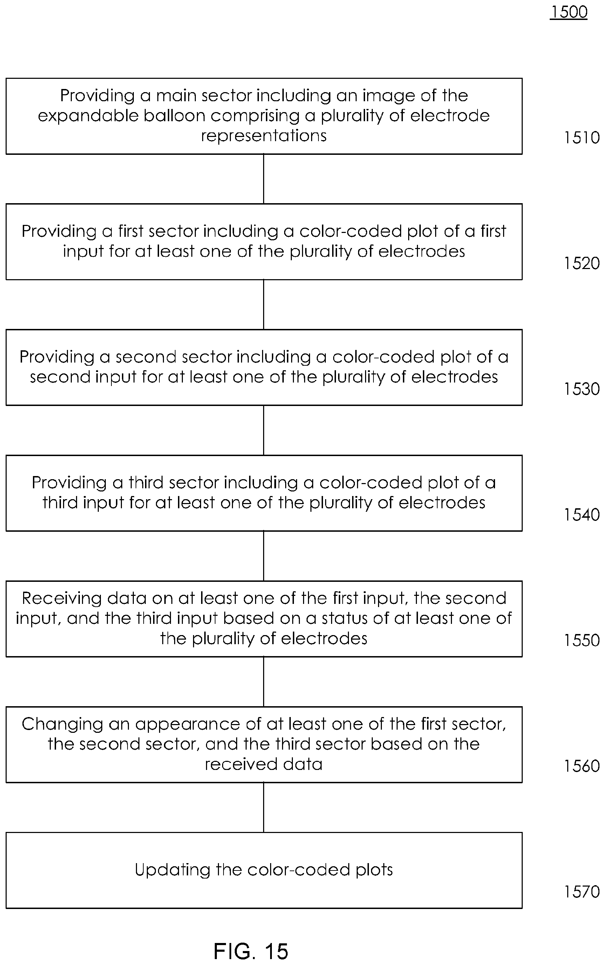

Figure 15 illustrates a method 1500 of using a graphical user interface (GUI) of a computer system. As described the computer system includes a processor and display to control a catheter including a balloon disposed thereon, the balloon including a surface and a plurality of electrodes disposed on the surface, the plurality of electrodes comprising a first electrode. Method 1500 includes providing a main sector including an image of the expandable balloon comprising a plurality of electrode representations including a first-electrode representation at step 1510. At step 1520, method 1500 includes providing a first sector including a color-coded plot of a first input for at least one of the plurality of electrodes. At step 1530, method 1500 includes providing a second sector including a color-coded plot of a second input for at least one of the plurality of electrodes. At step 1540, method 1500 includes providing a third sector including a color-coded plot of a third input for at least one of the plurality of electrodes. At step 1550, method 1500 includes receiving data on at least one of the first input, the second input, and the third input based on a status of at least one of the plurality of electrodes. At step 1560, method 1500 includes changing an appearance of at least one of the first sector, the second sector, and the third sector based on the received data corresponding to the first input, the second input, and the third input. At step 1570, method 1500 includes updating the color-coded plot of the first input for at least one of the plurality of electrodes, the second input for at least one of the plurality of electrodes, and the third input for at least one of the plurality of electrodes.

Method 1500 may include changing the image of the balloon. Further, method 1500 may include providing an alphanumeric indicator, color, shape indicator and/or otherwise changing the appearance of each of the plurality of electrode representations.

Method 1500 may include activating at least one of the plurality of electrodes.

The main sector color codes may be modified in method 1500 based on meeting a threshold of successful ablation configuration, based on falling under a threshold of successful ablation configuration, based on meeting a threshold of an on-going successful ablation configuration, based on falling under a threshold of an on-going successful ablation configuration, based on meeting a threshold of a completed successful ablation configuration, and based on falling under a threshold of a completed successful ablation configuration.

The first input may include one of temperature, impedance and impedance phase of one of the plurality of electrodes. The first input may be based on a derivative of input data, a comparison to the average of received data for each of the plurality of electrodes, and a threshold is utilized in compared first input.

The second input may include one of temperature, impedance and impedance phase of one of the plurality of electrodes. The second input includes first order data received by the processor, a derivative of the data received by the processor, a comparison of the received data for each of the plurality of electrodes and threshold is utilized in compared second input.

The third input includes one of temperature, impedance and impedance phase of one of the plurality of electrodes. The third input may include first order data received by the processor, a derivative of the data received by the processor, a comparison of the received data for each of the plurality of electrodes and a threshold is utilized in compared third input.

The appearance change of at least one of the first sector, the second sector, and the third sector may be based on the status of the ablation wherein the status of the ablation comprises one of pre-ablation processing, in-process ablation processing, and post-ablation processing. The appearance of each of the plurality of electrode is independently controlled.

Method 1500 further may include providing at least one image from a camera within the balloon.

Any of the examples or embodiments described herein may include various other features in addition to or in lieu of those described above. The teachings, expressions, embodiments, examples, etc., described herein should not be viewed in isolation relative to each other. Various suitable ways in which the teachings herein may be combined should be clear to those skilled in the art in view of the teachings herein.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but, in any order, as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometries, materials, dimensions, ratios, steps, and the like discussed above are illustrative. Accordingly, the claims should not be limited to the specific details of structure and operation set forth in the written description and drawings.

## Claims

1. An ablation system, comprising:
a catheter including an expandable portion disposed thereon, the expandable portion including a surface and a plurality of electrodes disposed on the surface, the plurality of electrodes comprising a first electrode; and
a graphical user interface (GUI) of a computer system, comprising:
a main sector including an image of the expandable balloon comprising a plurality of electrode representations including a first-electrode representation;
a first sector including a color-coded plot of a first input for at least one of the plurality of electrodes;
a second sector including a color-coded plot of a second input for at least one of the plurality of electrodes;
a third sector including a color-coded plot of a third input for at least one of the plurality of electrodes; and
a processor configured to:
receive data on at least one of the first input, the second input, and the third input based on a status of at least one of the plurality of electrodes,
change an appearance of at least one of the first sector, the second sector, and the third sector based on the received data corresponding to the first input, the second input, and the third input,
update the color-coded plot of the first input for at least one of the plurality of electrodes, the second input for at least one of the plurality of electrodes, and the third input for at least one of the plurality of electrodes.

2. The ablation system of claim 1, in which the processor is further configured to change a color of ones of the plurality of electrode representations, based on input values.

3. The ablation system of claim 1, in which at least one of the pluralities of electrode representations include an alphanumeric indicator, or a shape indicator.

4. The ablation system of claim 1, in which the processor is further configured to color code the main sector based on meeting a threshold of successful ablation configuration, falling under a threshold of successful ablation configuration, meeting a threshold of an on-going successful ablation configuration, falling under a threshold of an on-going successful ablation configuration, meeting a threshold of a completed successful ablation configuration, or falling under a threshold of a completed successful ablation configuration.

5. The ablation system of claim 1, wherein the first input is temperature associated with one of the plurality of electrodes.

6. The ablation system of claim 1, wherein the first input is one of temperature, impedance and impedance phase of one of the plurality of electrodes.

7. The ablation system of claim 1, wherein the first input is first order data received by the processor.

8. The ablation system of claim 1, wherein the first input is a derivative of the data received by the processor.

9. The ablation system of claim 1, wherein the first input comprises a comparison to the average of received data for each of the plurality of electrodes.

10. The ablation system of claim 9, wherein a first input threshold is utilized in compared first input.

11. The ablation system of claim 10, wherein the threshold comprises approximately 10%.

12. The ablation system of claim 1, wherein the second input comprises impedance associated with one of the plurality of electrodes.

13. The ablation system of claim 1, wherein the second input comprises one of temperature, impedance and impedance phase of one of the plurality of electrodes.

14. A graphical user interface (GUI) of a computer system, comprising:
a main sector including an image of the balloon comprising a plurality of electrode representations including a first-electrode representation;
a first sector including a color-coded plot of a first input for at least one of the plurality of electrodes;
a second sector including a color-coded plot of a second input for at least one of the plurality of electrodes;
a third sector including a color-coded plot of a third input for at least one of the plurality of electrodes; and
a processor configured to:
receive data on at least one of the first input, the second input, and the third input based on a status of at least one of the plurality of electrodes,
change an appearance of at least one of the first sector, the second sector, and the third sector based on the received data corresponding to the first input, the second input, and the third input,
update the color-coded plot of the first input for at least one of the plurality of electrodes, the second input for at least one of the plurality of electrodes, and the third input for at least one of the plurality of electrodes.
